# EUROPEAN PATENT APPLICATION

(11) **EP 2 979 697 A1**
(43) Date of publication of application: **03.02.2016**
(21) Application number: 14776456.7
(22) Date of filing: 25.03.2014
(51) Int. Cl.: A61K 31/404, A61K 9/14, A61K 9/62, A61K 47/10, A61K 47/26, A61K 47/32, A61K 47/34, A61K 47/36, A61K 47/38, A61P 13/02, A61P 13/08

(54) **ORAL ADMINISTRATION PREPARATION WITH MASKED BITTERNESS OF SILODOSIN**

(30) Priority: 26.03.2013 JP 2013065262; 30.08.2013 JP 2013180442
(71) Applicant: Kissei Pharmaceutical Co., Ltd., Matsumoto-shi, Nagano 399-8710 (JP)
(72) Inventor: SHIBATA, Yusuke, Azumino-shi Nagano 399-8304 (JP); ISSHIKI, Nobuyuki, Azumino-shi Nagano 399-8304 (JP); KIMURA, Shin-ichiro, Azumino-shi Nagano 399-8304 (JP)
(74) Representative: Boult Wade Tennant
(86) International application number: PCT/JP2014/058183
(87) International publication number: WO 2014/157137

(57) **Abstract**

The present invention provides a novel oral administration preparation that enables administration of silodosin, which is a drug with extremely strong bitterness, without a foreign-body sensation even without water, and has dissolution properties of being able to reproduce an effective blood concentration for the treatment of dysuria associated with benign prostatic hyperplasia or the like.

The present invention relates to a masked particle and a novel oral administration preparation comprising the masked particle or the like, wherein the masked particle obtained by granulating or coating a drug particle comprising a fine powder of silodosin with a coating agent comprising a non-enteric polymer, and a content of the non-enteric polymer is 80 parts by mass to 400 parts by mass relative to 100 parts by mass of silodosin.

## Description

### Technical Field

The present invention relates to a novel oral administration preparation that enables administration of silodosin, which is a drug with extremely strong bitterness, without a foreign-body sensation even without water, and has dissolution properties of being able to reproduce an effective blood concentration for the treatment of dysuria associated with benign prostatic hyperplasia or the like.

### Background Art

Silodosin is a medicament for dysuria having a selective urethral smooth muscle contraction inhibitory activity, and without causing a strong hypotensive activity (for example, see Patent literature 1) widely used as a pharmaceutical for the treatment of dysuria associated with benign prostatic hyperplasia. Capsules and tablets are used as pharmaceutical preparations comprising silodosin (for example, see Patent literatures 2 and 3), and these preparations have to be taken together with water. In recent years, development of a preparation that can be easily taken without water as a preparation readily to administer even for patients with swallowing difficulty such as the elderly has been required.

When a drug has bitterness, a chemical masking method such as a chemical modification and clathration of the drug itself (for example, see Patent literature 4), a sensual masking method by adding a sweetener, a flavor or the like (for example, see Patent literature 5 and Non-Patent literature 1), a physical masking method by coating the drug with a coating agent such as a gastro-soluble polymer or an enteric polymer (for example, see Patent literatures 6 to 9) or the like are known as a method of suppressing the bitterness (for example, see Non-Patent literature 2).

However, in the case of a drug with strong bitterness, the sensual masking method may not suppress bitterness sufficiently, and it is necessary to increase the coating amount of the coating agent in the physical masking method, and that causes a problem that the dissolution of the drug in the gastrointestinal tract decreases. Thus, it is difficult to combine the suppression of bitterness and dissolution properties in a variety of liquids.

Japanese unexamined publication 2008-231029 (Patent literature 6) discloses bitterness masked granules of rebamipide, wherein granules were made by spraying partially pregelatinized starch with rebamipide and methylcellulose and coating the granules with polyvinyl acetal diethylamino acetate, a gastro-soluble polymer.

Japanese unexamined publication 2005-513008 (Patent literature 7) discloses coated granules of fexofenadine obtained by subjecting a mixture of fexofenadine and precipitated silica to granulation using Eudragit (registered trademark) E100 and further coating the granules with a polymer dispersion of Eudragit (registered trademark) E100 comprising precipitated silica.

WO2008/01871 (Patent literature 8) discloses granules obtained by spraying aminoalkyl methacrylate copolymer E on a mixture of mitiglinide and microcrystalline cellulose.

Japanese unexamined publication 2007-63263 (Patent literature 9) discloses amlodipine-comprising particles obtained by spraying a coating solution comprising aminoalkyl methacrylate copolymer E on a mixture of amlodipine besylate and light anhydrous silicic acid.

However, in any of these literatures, any preparations with both of suppression of bitterness of silodosin and dissolution properties in a variety of liquids that can be taken even without water are not described, and development of new preparations has been desired.

Non-patent literature 1: PHARM TECH JAPAN, 2007, Vol.23, pp.1413-1417
Non-patent literature 2: PHARM TECH JAPAN, 2012, Vol.28, No.2, Extra edition, edited by PLCM (KOUYAKU) "subetegawakaru koukuunai houkaijou handbook*"*
Patent literature 1: Japanese unexamined publication H06-220015
Patent literature 2: International Publication No. WO2004/054574 pamphlet
Patent literature 3: Japanese unexamined publication 2008-44960
Patent literature 4: Japanese unexamined publication 2008-44870
Patent literature 5: Japanese unexamined publication 2008-94837
Patent literature 6: Japanese unexamined publication 2008-231029
Patent literature 7: Japanese unexamined publication 2005-513008
Patent literature 8: International Publication No. WO2008/01871 pamphlet
Patent literature 9: Japanese unexamined publication 2007-63263

### Disclosure of the Invention

### Problems that the Invention aims to solve

The problem of the present invention is to provide a novel oral administration preparation that enables administration of silodosin, which is a drug with an extremely strong bitterness, without a foreign-body sensation even without water, and has dissolution properties of being able to reproduce an effective blood concentration for the treatment of dysuria associated with benign prostatic hyperplasia or the like.

### Means to solve the Problem

In terms of making the oral administration preparation of the present invention, on the characteristics of silodosin, there were various problems to be overcome. First, silodosin has a chemical nature of easily decomposing by a filler or the like widely used as a pharmaceutical additive. Also, it requires a lot of coating agent for suppressing the bitterness since it has an extremely strong bitterness and is a substance of a needle-like crystal. Therefore, there are problems that a dissolution property decreases or a foreign-body sensation remains when it was taken without water. Moreover, the preparation must be able to properly reproduce the blood concentration where silodosin preparation already used as a medicament for dysuria associated with benign prostatic hyperplasia exhibits efficacy.

The present inventors have studied earnestly to solve the above problems while overcoming these points. It was not able to mask the strong bitterness of silodosin, for example, by the sensual masking method such as cocoa powder, calcium lactate or the like, or the chemical masking method such as carrageenan or the like. It was found that an enteric base (an enteric polymer) used generally in the physical masking method causes incompatibility with silodosin and are unusable. As a result of further various investigations, surprisingly, it was found that an oral administration preparation, which exhibits highly desirable properties such that it can be taken without feeling a strong bitterness even without water and achieve effective blood concentrations in human by the desired dissolution properties, can be obtained by using the masked particle of the present invention. Based on these findings, the present invention has been accomplished.

That is, the present invention relates to:
[1] a masked particle obtained by granulating or coating drug particle comprising a fine powder of silodosin with a coating agent comprising a non-enteric polymer, wherein a content of the non-enteric polymer is 80 parts by mass to 400 parts by mass relative to 100 parts by mass of silodosin;
[2] the masked particle as described in the above [1], wherein a dissolution rate after 15 minutes at pH 6.8 of an oral administration preparation comprising the masked particle is more than 85%;
[3] the masked particle as described in the above [1] or [2], wherein a time to start feeling bitterness in human bitterness sensory test for an oral administration preparation comprising the masked particle is more than 30 seconds;
[4] the masked particle as described in any one of the above [1] to [3], wherein the drug particle comprising a fine powder of silodosin is a mixture of silodosin and an additive;
[5] the masked particle as described in the above [4], wherein the drug particle comprising a fine powder of silodosin is a granule of silodosin and an additive;
[6] the masked particle as described in the above [4] or [5], wherein the additive is at least one additive selected from the group consisting of a sugar or a sugar alcohol and a starches;
[7] the masked particle as described in any one of the above [1] to [6], wherein the non-enteric polymer is a gastro-soluble polymer;
[8] the masked particle as described in any one of the above [1] to [6], wherein the non-enteric polymer is ethylcellulose, polyvinylacetal diethylaminoacetate or aminoalkyl methacrylate copolymer E;
[9] the masked particle as described in any one of the above [1] to [8,] wherein a content of the non-enteric polymer is 100 parts by mass to 200 parts by mass relative to 100 parts by mass of silodosin;
[10] the masked particle as described in any one of the above [1] to [9], wherein a content of silodosin in the masked particles is 5 to 25 mass%;
[11] the masked particle as described in any one of the above [1] to [10], wherein a content of the non-enteric polymer in the masked particle is 15 to 30 mass%;
[12] the masked particle as described in any one of the above [1] to [10], wherein a content of the non-enteric polymer is 20 parts by mass to 40 parts by mass relative to 100 parts by mass of the drug particle;
[13] an oral administration preparation comprising the masked particle as described in any one of the above [1] to [12];
[14] the oral administration preparation comprising the masked particle as described in the above [13], wherein a dosage form is a tablet;
[15] a method for the preparation of a masked particle, comprising the steps of:
   (a) preparing a drug particle by mixing or granulating a fine powder of silodosin and an additive, and
   (b) preparing a masked particle by granulating or coating a drug particle obtained by the step (a) with a coating agent comprising a non-enteric polymer, wherein a content of the non-enteric polymer is 80 parts by mass to 400 parts by mass relative to 100 parts by mass of silodosin; and the like.

In the present invention, the term "non-enteric polymer" means a water-insoluble polymer other than an enteric polymer, and for example, a gastro-soluble polymer or a water-insoluble polymer can be illustrated. As the gastro-soluble polymer, for example, methyl methacrylate-butyl methacrylate-dimethylaminoethyl methacrylate copolymer such as aminoalkyl methacrylate copolymer E (for example, Eudragit (registered trademark) EPO, Eudragit (registered trademark) E100) and the like, methyl methacrylate-diethylaminoethyl methacrylate copolymer (for example, Kollicoat (registered trademark) smartseal 30D), gastro-soluble polyvinyl derivatives such as polyvinyl acetal diethylamino acetate (for example, AEA (registered trademark)) and the like, and the like can be illustrated.

As the water-insoluble polymer, for example, water-insoluble acrylic acid copolymer such as ethyl acrylate-methyl methacrylate copolymer such as ethyl acrylate-methyl methacrylate copolymer dispersion liquid (for example, Eudragit (registered trademark) NE30D) and the like, ethyl acrylate-methyl methacrylate-trimethyl ammonium ethyl methacrylate chloride copolymer such as aminoalkyl methacrylate copolymer RS (for example, Eudragit (registered trademark) RS100, Eudragit (registered trademark) RSPO, Eudragit (registered trademark) RL, Eudragit (registered trademark) RLPO), and amino alkyl methacrylate copolymer RS aqueous dispersion (for example, Eudragit (registered trademark) RS30D, Eudragit (registered trademark) RL30D)) and the like, water-insoluble cellulose ether such as ethylcellulose (for example, Ethocel (registered trademark)), ethylcellulose aqueous dispersion (for example, Aquacoat (registered trademark)) and the like, vinyl acetate resin (for example, Kollicoat (registered trademark) SR, Kollicoat (registered trademark) SR30D) and the like can be illustrated. The non-enteric polymer is preferably ethylcellulose or a gastro-soluble polymer, more preferably ethylcellulose, methyl methacrylate-diethylaminoethyl methacrylate copolymer, aminoalkyl methacrylate copolymer E or polyvinyl acetal diethylamino acetate, and more preferably ethylcellulose, aminoalkyl methacrylate copolymer E or polyvinyl acetal diethylamino acetate, more preferably ethylcellulose or aminoalkyl methacrylate copolymer E, more preferably aminoalkyl methacrylate copolymer E. These non-enteric polymers may be used in a combination of two or more, if necessary.

A coating agent comprising the non-enteric polymer used in the present invention may include an additive, a water-soluble polymer or the like in addition to the above non-enteric polymer, if necessary. When the water-soluble polymer is used, the ratio of the mass of the water-soluble polymer relative to the total mass of the non-enteric polymer and the water-soluble polymer is preferably not more than 20%. As the additive, for example, plasticizers, lubricants, surfactants and the like can be illustrated. As the plasticizers, for example, stearic acid, triacetin, triethyl citrate, macrogol, glycerin, glycerin fatty acid esters, castor oil, diethyl sebacate, dibutyl sebacate and the like can be illustrated. As the lubricants, for example, talc, stearic acid, magnesium stearate, calcium stearate and the like can be illustrated. As the surfactants, for example, sodium lauryl sulfate, polysorbate and the like can be illustrated. As the water-soluble polymer, for example, hypromellose, methylcellulose, hydroxypropylcellulose, polyvinyl alcohol, povidone, carmellose sodium, sodium alginate and the like can be illustrated. These additives and the water-soluble polymer can be also used in combination of two or more.

In the present invention, as the content of the non-enteric polymer, for example, 80 to 400 parts by mass, 80 to 300 parts by mass, 80 to 200 parts by mass, 100 to 400 parts by mass, 100 to 300 parts by mass, 100 to 200 parts by mass and the like relative to 100 parts by mass of silodosin can be illustrated, and 100 to 200 parts by mass is preferable.

The content of the non-enteric polymer in the masked particle of the present invention is preferably 15 to 30 mass%, and more preferably 15 to 25 mass%.

In the present invention, the content of the non-enteric polymer, for example, is generally 20 to 50 parts by mass relative to 100 parts by mass of the drug particle, preferably 20 to 40 parts by mass, and more preferably 30 to 40 parts by mass.

In the present invention, the "average particle diameter" means the 50% particle diameter (mass-based median size). This 50% particle diameter can be measured with a particle distribution measuring sifter (for example, a robot shifter RPS-205 Model, manufactured by Seishin Enterprise Co., Ltd.).

The average particle diameter of the masked particle of the present invention, for example, is generally not more than 300 µm, and preferably about 100 to 250 µm.

Silodosin used in the present invention can be also commercially available, or can be also prepared by methods described in literatures (for example, see Patent literature 1) or a similar method thereto.

"Fine powder of silodosin" used in the present invention may be particles without an agglomerate mass, and may be crushed or grinded or the like, if necessary. The average particle diameter of the fine powder of silodosin is preferably not more than about 50 µm, and more preferably about 1 to 30 µm.

As "drug particle comprising a fine powder of silodosin" used in the present invention, it is desirable to use an appropriate additive in addition to silodosin, for example, a mixture of silodosin and an appropriate additive, a granulated material of silodosin and an appropriate additive, an appropriate additive coated with silodosin and the like can be illustrated.

As the additive used in the drug particle, various additives without causing incompatibility between silodosin such as disintegrants, fillers, binders, lubricants, sweeteners, sour agents, foaming agents, flavors, colorants and the like can be appropriately used. As the disintegrants, for example, low-substituted hydroxypropylcellulose, croscarmellose sodium, carmellose calcium, carmellose sodium, rice starch, corn starch, potato starch, sodium carboxymethyl starch, carmellose, partially pregelatinized starch, pregelatinized starch, crospovidone, microcrystalline cellulose and the like can be illustrated. As the fillers, for example, rice starch, corn starch, potato starch, partially pregelatinized starch, pregelatinized starch, trehalose, microcrystalline cellulose, magnesium aluminometasilicate, anhydrous calcium phosphate, precipitated calcium carbonate, calcium silicate, calcium lactate, lactose, fructose, D-mannitol, erythritol, xylitol, maltose, D-sorbitol, maltitol and the like can be illustrated. As the binders, for example, starches, microcrystalline cellulose, hydroxypropylcellulose, hypromellose, povidone, dextrin, gelatin, pullulan, polyvinyl alcohol, sodium alginate, polyethylene glycol and the like can be illustrated. As the lubricants, for example, magnesium stearate, calcium stearate, stearic acid, talc, light anhydrous silicic acid, sucrose fatty acid esters, sodium stearyl fumarate, polyethylene glycols, glycerin monostearate and the like can be illustrated. As the sweeteners, for example, aspartame, saccharin, saccharin sodium, dipotassium glycyrrhizinate, stevia, thaumatin, acesulfame potassium, sucralose and the like can be illustrated. As the sour agents, for example, citric acid, tartaric acid, malic acid, ascorbic acid and the like can be illustrated. As the foaming agents, for example, sodium hydrogen carbonate, sodium carbonate, calcium carbonate and the like can be illustrated. As the flavoring agents, for example, L-aspartic acid, sodium chloride, magnesium chloride, sodium citrate, calcium citrate, L-sodium glutamate, sodium hydrogen carbonate and the like can be illustrated. As the flavors, for example, strawberry, yogurt, banana, pineapple, orange, lemon, menthol, peach, apple, chocolate, cocoa, vanilla, tea, green tea and the like can be illustrated. As the colorants, for example, food dyes such as food yellow 5, food red 2, food blue 2 and the like, yellow ferric oxide, red ferric oxide, caramel dye, titanium oxide and the like can be illustrated.

As the additive used in the drug particle in the present invention, as the fillers or disintegrants, for example, sugars or sugar alcohols and starches and the like are preferable, and starches are more preferable. As the sugars or sugar alcohols, for example, D-mannitol, erythritol, xylitol, maltose, D-sorbitol, maltitol and the like can be illustrated, and D-mannitol is more preferable. As the starches, for example, corn starch, rice starch, potato starch, partially pregelatinized starch, pregelatinized starch and the like can be illustrated, and partially pregelatinized starch and pregelatinized starch are more preferable. As the lubricants, for example, magnesium stearate, calcium stearate, talc and the like are preferable and talc is more preferable. As the binders, for example, starches, hydroxypropylcellulose, hypromellose, povidone, dextrin, gelatin, pullulan, polyvinyl alcohol, sodium alginate, polyethylene glycol and the like are preferable, and hydroxypropylcellulose and hypromellose are more preferable. These additives may be used in combination of two or more, if necessary.

The content of silodosin in the masked particle of the present invention is preferably 30 mass% or less, more preferably 5 to 25 mass%, and more preferably 5 to 16 mass%.

The content of silodosin in the drug particle used in the present invention is preferably 50 mass% or less, for example, 10 to 40 mass%, 10 to 30 mass%, 20 to 27 mass% and the like.

### (Preparing method of the masked particle)

The masked particle of the invention can be prepared by a method generally used in manufacturing the masked particle such as the core granules coating method, granulation matrix method, granulation coating method and the like. For example, it can also be prepared by granulating or coating the drug particle obtained by mixing or granulating an additive and silodosin with a coating agent comprising a non-enteric polymer. In these series of manufacturing, as the method of granulating or coating, a high shear granulating method, a tumbling fluidized bed granulating method, a fluidized bed granulating method and the like can be illustrated, and a fluidized bed granulating method is preferable.

Specifically, for example, in the core granules coating method, the masked particle can be also prepared by sequentially coating core particles such as commercial or granulated microcrystalline cellulose, D-mannitol, corn starch, magnesium hydroxide, magnesium carbonate, sucrose and the like with a dispersion liquid comprising silodosin, and a solution or dispersion liquid of the coating agent comprising the non-enteric polymer or by coating core particles with these mixed solution.

Also, for example, in the granulation matrix method, the masked particle can be also prepared by granulating or coating a mixture of silodosin and an additive (for example, D-mannitol, partially pregelatinized starch, pregelatinized starch, light anhydrous silicic acid and the like) while spraying a solution or dispersion liquid of the coating agent comprising the non-enteric polymer.

Further, for example, in the granulation coating method, the masked particle can be also prepared by granulating a mixture of silodosin and an additive (for example, D-mannitol, partially pregelatinized starch, pregelatinized starch, light anhydrous silicic acid and the like) while spraying a solution of a water-soluble binder, and then coating the obtained granule while spraying a solution or dispersion liquid of the coating agent comprising the non-enteric polymer. As the water-soluble binder, hydroxypropylcellulose and hypromellose are preferable.

In the above methods, the granulation matrix method or the granulation coating method is preferable, and the granulation coating method is more preferable.

The solvent used to dissolve or disperse the non-enteric polymer is not particularly limited, for example, alcohols such as methanol, ethanol, isopropyl alcohol and the like, acetone, toluene, methyl ethyl ketone and water, or a mixed solvent thereof and the like can be illustrated, ethanol and water are preferable, and water is more preferable. Although aminoalkyl methacrylate copolymer E is insoluble in water, it can be also used as an aqueous solution dissolved in water in an acidic (pH 5 or less) or as an aqueous dispersion mixed aminoalkyl methacrylate copolymer E with sodium lauryl sulfate and at least one plasticizer selected from stearic acid, diethyl sebacate and dibutyl sebacate in any ratio.

The masked particle of the present invention, to prevent the agglomeration during manufacture, further may be used by overcoating with a suitable additive, and also include these overcoated masked particle. With the proviso that, in the present specification, the mass of the masked particle is not included in the mass of the additive used in the overcoat. As the additive used in the overcoat, for example, sugars such as lactose, glucose, sucrose, fructose and the like, sugar alcohols such as D-mannitol, erythritol, xylitol, maltose, D-sorbitol, maltitol and the like can be illustrated, and D-mannitol is preferable.

A method to overcoat is not particularly limited. For example, the overcoated masked particle can be also prepared by coating the masked particle of the present invention while spraying an aqueous solution of the additive (for example, sugars or sugar alcohols). The content of the additive used in the overcoat is generally 1 to 20 parts by mass, preferably 2 to 15 parts by mass, and more preferably 5 to 10 parts by mass, relative to 100 parts by mass of the masked particles.

### (Oral Administration Preparation)

An oral administration preparation of various dosage forms can be prepared by using the masked particle of the present invention. As the dosage form of the oral administration preparation of the present invention, for example, granules, powders, tablets and the like can be illustrated.

The oral administration preparation of the present invention can be prepared using the masked particle of the present invention and a pharmaceutical additive commonly used in an orally rapidly disintegrating preparation in accordance with conventional methods in the pharmaceutical field.

For example, in the case of tablets, the oral administration preparation can be prepared by tableting the masked particle of the present invention with a pharmaceutical additive commonly used in an orally rapidly disintegrating preparation, by a known method such as a direct powder compression method (a direct tableting method), granulating method and the like or a similar method thereto.

Specifically, for example, in the direct tableting method, the oral administration preparation can be also prepared by mixing a mixture comprising the masked particle of the present invention and a pharmaceutical additive such as fillers, disintegrants, binders, lubricants and the like without granulating using a mixer, and tableting. Also, for example, in the granulating method, not only the orally administration preparation can be also prepared by granulating a mixture of fillers, disintegrants and the like using water, a mixed solution of water and ethanol, or a solution or suspension of binders or disintegrants and the like, further mixing the granules with the masked particle of the invention, lubricants and the like using a mixer, and tableting, but also the orally administration preparation can be also prepared by granulating a mixture of the masked particle of the invention, fillers, disintegrants and the like using water, a mixed solution of water and ethanol, or a solution or suspension of binders or disintegrants and the like, further mixing it with the addition of lubricants using a mixer, and tableting.

Also, in granules, the orally administration preparation can be also prepared by performing a fluidized bed granulation in accordance with the granulating method of the tablet or performing the agitation granulation. Powders or the like can also prepared by mixing a pharmaceutical additive in accordance with the direct tableting method of tablet.

As the pharmaceutical additive commonly used in an orally rapidly disintegrating preparation, the above additive used in the above drug particle can be used. As the disintegrants, partially pregelatinized starch, crospovidone, low-substituted hydroxypropylcellulose, carmellose calcium, carmellose sodium, corn starch and the like are preferable. As the fillers, sugar alcohols such as D-mannitol, erythritol, xylitol, maltose, D-sorbitol, maltitol and the like, corn starch, microcrystalline cellulose and the like are preferable. As the lubricants, sodium stearyl fumarate, calcium stearate, talc, light anhydrous silicic acid and the like are preferable. These pharmaceutical additives may be used in combination of two or more, if necessary.

### (Production Example of Oral Administration Preparation)

The following describes the method of manufacturing the oral administration preparation of the present invention. However, it is not limited thereto.

### [Production Example 1]

For example, powders can be also prepared by mixing the masked particle of the present invention with at least one pharmaceutical additive selected from sugars such as lactose, fructose and the like, sugar alcohols such as D-mannitol, erythritol, xylitol and the like, starches such as rice starch, corn starch, potato starch, partially pregelatinized starch and the like, microcrystalline cellulose and crospovidone using a mixer. In the above mixing step, one or two or more of fillers, disintegrants, binders, lubricants, foaming agents, sweeteners, flavoring agents, fluidizers, flavors, colorants or the like may be further added in combination, if necessary.

### [Production Example 2]

For example, tablets can be also prepared by mixing the masked particle of the present invention with at least one pharmaceutical additive selected from sugars such as lactose, fructose and the like, sugar alcohols such as D-mannitol, erythritol, xylitol and the like, starches such as rice starch, corn starch, potato starch, partially pregelatinized starch and the like, microcrystalline cellulose, crospovidone, sodium stearyl fumarate, calcium stearate, talc and light anhydrous silicic acid using a mixer, and tableting. In the above mixing step, one or two or more of fillers, disintegrants, binders, lubricants, foaming agents, sweeteners, flavoring agents, fluidizers, flavors, colorants or the like may be further added in combination, if necessary.

### [Production Example 3]

For example, granules (1) can be also prepared by mixing at least one pharmaceutical additive selected from sugars such as lactose, fructose and the like, sugar alcohols such as D-mannitol, erythritol, xylitol, maltose, D-sorbitol, maltitol and the like, starches such as corn starch, rice starch, potato starch, partially pregelatinized starch, pregelatinized starch and the like, and microcrystalline cellulose, and granulating the mixture while spraying a solution or dispersion liquid of partially pregelatinized starch or crospovidone. In the above mixing and granulating steps, a high shear granulating method, a tumbling fluidized bed granulating method, a fluidized bed granulating method and the like can be used, and a fluidized bed granulating method is preferable. Then, tablets can be also prepared by mixing the masked particle of the present invention and the above granules (1) with at least one lubricant selected from sodium stearyl fumarate, calcium stearate, talc and light anhydrous silicic acid using a mixer, and tableting. In the above mixing step, one or two or more of fillers, disintegrants, binders, lubricants, foaming agents, sweeteners, flavoring agents, fluidizers, flavors, colorants or the like may be further added in combination, if necessary.

### [Production Example 4]

For example, granules (2) can be also prepared by mixing at least one pharmaceutical additive selected from sugars such as lactose, fructose and the like, sugar alcohols such as D-mannitol, erythritol, xylitol, maltose, D-sorbitol, maltitol and the like, starches such as corn starch, rice starch, potato starch, partially pregelatinized starch, pregelatinized starch and the like and microcrystalline cellulose, and granulating the mixture while spraying water or a mixed solution of water and ethanol. The above mixing and granulating steps can be used by a high shear granulating method, a tumbling fluidized bed granulating method, a fluidized bed granulating method and the like, and a fluidized bed granulating method is preferable. Then, tablets can be also prepared by mixing the masked particle of the present invention and the above granules (2) with at least one lubricant selected from sodium stearyl fumarate, calcium stearate, talc and light anhydrous silicic acid using a mixer, and tableting. In the above mixing step, one or two or more of fillers, disintegrants, binders, lubricants, foaming agents, sweeteners, flavoring agents, fluidizers, flavors, colorants or the like may be further added in combination, if necessary.

### [Production Example 5]

For example, granules can be also prepared by mixing the masked particle of the present invention with the above granules (1) or the above granules (2) using a mixer. In the above mixing step, one or two or more of fillers, lubricants, foaming agents, sweeteners, flavoring agents, fluidizers, flavors, colorants or the like may be further added in combination, if necessary.

### [Production Example 6]

For example, granules (3) can be also prepared by mixing a mixture of D-mannitol and microcrystalline cellulose using a fluidized bed granulation dryer, granulating the mixture while spraying an aqueous dispersion of crospovidone, and then sizing using a mill. Then, tablets can be also prepared by mixing the masked particle of the present invention and the above granules (3) with sodium stearyl fumarate using a mixer, and tableting. In the above mixing step, one or two or more of fillers, lubricants, foaming agents, sweeteners, flavoring agents, fluidizers, flavors, colorants or the like may be further added in combination, if necessary.

### [Production Example 7]

For example, granules (4) can be also prepared by mixing a mixture of D-mannitol, microcrystalline cellulose and crospovidone using a fluidized bed granulation dryer, granulating the mixture while spraying water, and then sizing using a mill. Then, tablets can be also prepared mixing the masked particle of the present invention and the above granules (4) with sodium stearyl fumarate using a mixer, and tableting. In the above mixing step, one or two or more of fillers, lubricants, foaming agents, sweeteners, flavoring agents, fluidizers, flavors, colorants or the like may be further added in combination, if necessary.

### [Production Example 8]

In the above Production Example 6 or 7, tablets can be also prepared by the similar method as described in Production Example 6 or 7, using corn starch instead of microcrystalline cellulose and using partially pregelatinized starch instead of crospovidone.

In the preparing step of the masked particle or oral administration preparation of the present invention, "granulating", "coating", "mixing", "tableting" may be performed using conventional methods in the pharmaceutical technical field. In the "granulating" and "coating", for example, a fluidized bed granulator, a tumbling fluidized bed granulating method, a high shear granulator or the like can be also used. In the "mixing", for example, a V type mixer, a Bohle container or the like can be also used. In the "tableting", for example, a single punch tableting machine, a rotary tableting machine or the like can be also used. The tableting pressure is, for example, 1 to 20 kN, and preferably 2 to 15 kN.

The masked particle of the present invention and an oral administration preparation comprising the same exhibits a good bitterness-masking effect. The bitterness-masking effect can be quantitatively evaluated by the bitterness sensory test as described below. An average score of the bitterness score is preferably 2 or less, and more preferably 1 or less. In addition, the time to start feeling bitterness is preferably 30 seconds or more and more preferably a longer time such as 40 seconds or more or 50 seconds or more.

The oral administration preparation of the present invention exhibits a good dissolution property independent on the pH in the gastrointestinal tract. That is, the oral administration preparation of the present invention shows 80% or more drug dissolution rates after 15 minutes in the 1st fluid (pH of about 1.2) and the 2nd fluid (pH of about 6.8) of the dissolution test in the Japanese Pharmacopoeia. The dissolution rate is preferably 85% or more after 15 minutes in the 1st fluid (pH of about 1.2) of the dissolution test in the Japanese Pharmacopoeia, and more preferably 85% or more after 15 minutes in the 2nd fluid (pH of about 6.8) of the dissolution test in the Japanese Pharmacopoeia as well. Dissolution properties can be quantitatively evaluated by a dissolution test as described below.

The oral administration preparation of the present invention disintegrates preferably in a short time in the mouth in order to be taken without water. For example, in the oral disintegration test as described below, the mean oral disintegration time may be generally adjusted to 60 seconds or less, preferably 40 seconds or less, and more preferably 30 seconds or less.

The oral administration preparation of the present invention preferably has an appropriate hardness for the convenience of manufacture and transport and the like. For example, the hardness may be generally adjusted to 20 N or more, preferably 30 N or more, and more preferably 40 N or more in the hardness test as described below.

The oral disintegration time and hardness can be also adjusted by appropriately selecting the type and amount of pharmaceutical additives, production methods (for example, a granulating method or the like), production conditions (for example, a tableting pressure or the like) and the like.

In the oral administration preparation of the present invention, the content of silodosin per unit preparation is generally 2 to 8 mg and preferably 2 mg, 4 mg or 8 mg. In the oral administration preparation of the present invention, for example, in a case of tablet, 50 to 500 mg, 50 to 300 mg, 100 to 250 m0g, 100 to 200 mg and the like can be illustrated as a mass per tablet, and 0.4 to 16% can be illustrated as a content ratio of silodosin therein. In a case of granules, powders or the like, 200 to 3000 mg, 500 to 2000 mg, 500 to 1000 mg and the like can be illustrated as a mass per one dose, and 0.06 to 4% can be illustrated as a content ratio of silodosin therein.

When the oral administration preparation of the present invention are employed in the practical treatment, the dosage of the active ingredient is appropriately decided depending on the sex, age, body weight, degree of disorders of each patient and the like, and for example, it can be administrated approximately within the range of 1 to 16 mg per day for an adult. Preferably, it is orally administered by dividing 2 to 8 mg into once or twice a day for an adult.

### Effect of the Invention

The masked particle of the present invention is pharmaceutically stable and can suppress an extremely strong bitterness of silodosin. It also has a rapid dissolution property similar to commercially available tablets of silodosin (URIEF (registered trademark) tablets). Therefore, it can be used for the oral administration preparations, which can be easily taken without a foreign-body sensation even without water. Also the oral administration preparation of the present invention suppresses the specific bitterness of silodosin, and has a rapid dissolution property and bioequivalence similar to commercially available tablets of silodosin (URIEF (registered trademark) tablets). Therefore, it is useful as silodosin-comprising preparations which can be taken without a foreign-body sensation even without water.

### Best Modes for Carrying out the Invention

The contents of the present invention are further described in more detail by the following Test Examples, Examples and Comparative Examples. However, the content of the present invention is not limited thereto.

### Examples

### Test Example 1

### Bitterness Sensory Test

In the 1 to 3 healthy male, each of about 200 mg of masked particles prepared in Examples 1 to 3 and Comparative Example 1 or 1 tablet prepared in Examples 4 to 9 was put in the mouth. Bitterness was scored according to Table 1 when they start feeling bitterness, and an average was calculated. For the tablet, the tablet was put in the mouth, and disintegrated in the mouth while gently rolling by a tongue, and the time to start feeling bitterness was also evaluated.

**[Table 1]**

| | |
|---|---|
| 0 | None |
| 1 | Almost none |
| 2 | Slightly bitter |
| 3 | Bitter |
| 4 | Extremely bitter |

### Test Example 2

### Oral disintegration Test

In the 1 to 3 healthy male, each tablet prepared in test preparation and Examples 4 to 11 was put in the mouth. The tablet was disintegrated in the mouth while gently rolling lightly by a tongue, and the time required to disintegrate the tablet in the mouth was measured, and an average was calculated.

### Test Example 3

### Hardness Test

The hardness of a test preparation and tablet prepared in Examples 5 to 11 was measured using a hardness meter (PC-30, Okada Seiko Co., Ltd.).

### Test Example 4

### Dissolution Test Method 1

The dissolution test was performed for the granules prepared in Examples 1 to 3 and Comparative Example 1 at a paddle rotation speed of 50 rpm using the 2nd fluid of the dissolution test in the Japanese Pharmacopoeia as a test fluid, according to the paddle method in the dissolution test method described in the Japanese Pharmacopoeia, 16th edition. Dissolution rates after 15 minutes were measured using an ultraviolet absorption photometer. In addition, the test for each preparation was performed twice, and the dissolution rate was calculated as the average score.

### Detector: Ultraviolet absorption photometer (wavelength: 270 nm and 350 nm)

### Test Example 5

### Dissolution Test Method 2

The dissolution test was performed for a test preparation and the tablets prepared in Examples 4 to11 at a paddle rotation speed of 50 rpm using the 2nd fluid of the dissolution test in the Japanese Pharmacopoeia as a test fluid, according to paddle method of the dissolution test method described in the Japanese Pharmacopoeia, 16th edition. Dissolution rate was calculated by quantifying silodosin in sampling solution by high-performance liquid chromatography method. In addition, the test for each of preparation was performed using randomly selected 2 to 3 samples, and the dissolution rate was calculated as the average score.

### Detector: Ultraviolet absorption photometer (wavelength: 270 nm)

### Test Example 6

### Dissolution Test Method 3

The dissolution test was performed using the 1st fluid of the dissolution test in the Japanese Pharmacopoeia as a test fluid in the similar manner as in Test Example 5 and dissolution rate was calculated. Accordingly, the dissolution rate after 15 minutes of the test preparation was 102.1%.

### Test Example 7

### Measurement of Particle Distribution

A particle distribution was measured to determine a 50% particle diameter (mass-based median size) by sifting, using a robot sifter (RPS-205 model, Seishin Enterprise Co., Ltd.).

### Test Example 8

### Compatibility Test

Silodosin and a variety of polymer-based materials were mixed in the mass ratio of 1:1. The mixtures were stored at 40°C under 75% relative humidity for 4 weeks, and the changes on blending, i.e. incompatibility, were checked. Degradation products were quantified by a liquid chromatography method and a degree of discoloration was checked by visual examination.

### Quantitation Method

Samples equivalent to 20 mg of silodosin were weighed, and test solutions were prepared and analyzed by the liquid chromatography method. Each peak area was measured by an automatic integration method and a content rate of degradation product (KMD-3241) (%) was calculated by an area percentage method.

### Detector: Ultraviolet absorption photometer (wavelength: 225 nm)

**[Table 2]**

| Polymer-based materials | | Color change (fading) | Degradation products(%) |
|---|---|---|---|
| Silodosin(reference) | | - | 0.04 |
| Hypromellose 60SH50 | water-soluble polymer | + | 0.06 |
| Sodium Alginate | water-soluble polymer | + | 0.07 |
| Aminoalkylmethacrylate copolymer RS (EUDRAGIT (registered trademark) RS) | water-insoluble polymer | - | 0.06 |
| Aminoalkylmethacrylate copolymer RS (EUDRAGIT (registered trademark) RL) | water-insoluble polymer | - | 0.09 |
| Ethylcellulose | water-insoluble polymer | - | 0.05 |
| Aminoalkylmethacrylate copolymer E | gastro-soluble polymer | + | 0.06 |
| Methacrylic acid copolymer L | enteric polymer | +++ | 0.43 |
| Dry methacrylic acid copolymerLD | enteric polymer | +++ | 0.24 |
| Hydroxypropylmethylcelluloseacetate AS-MF | enteric polymer | + | 0.51 |

| | | | |
|---|---|---|---|
| -: no change +: slight change ++: change +++: remarkable change | | | |

As shown in Table 2, hydroxyproplymethylcellulose acetate succinate AS-MF remarkably increases in degradation products and was incompatible. In methacrylic acid copolymer L and dry methacrylic acid copolymer LD, colors were changed and degradation products remarkably increased. They were incompatible.

### Test Example 9

### Bioequivalence Test

### (1) Test Method

In healthy adult male, the bioequivalence of both preparations was evaluated by measuring plasma silodosin concentration in cases of orally administrating a single dose of the test preparation comprising 4 mg of silodosin without water with disintegrating in the oral cavity, and orally administrating a single dose of commercially available tablet comprising 4 mg of silodosin (a standard preparation) with water, in the fasting state.

### (2) Test Drug

Commercial URIEF (registered trademark) 4 mg tablet was used as the standard preparation. A tablet of a formulation shown in Table 3 was prepared according to the method of Example 10 as the test preparation.

**[Table 3]**

| Components | | | Amounnt of components in each tablet (mg) |
|---|---|---|---|
| Masked particles | Drug | Silodosin | 4 |
| | Additive | Partially pregelatinized starch | 14.72 |
| | | Talc | 3.45 |
| | | Hydroxypropylcellulose | 0.28 |
| | | Aminoalkylmethacrylate copolymer E | 7 |
| | | Sodium lauryl sulfate | 0.71 |
| | | acid | 1.05 |
| Pharmaceutical additive | | D-Mannitol, Microrystalline cellulose, Crospovidone, Corn starch, Sodium stearyl fumarate, colorant, sweetened, flavor | 169.4 |
| | | Total | 200.6 |

### (3) Endpoint

As the bioequivalence endpoint, AUC₀₋₄₈ (Area under the plasma concentration-time curve over 48 hours after administrating the test drug) and Cmax (Maximum plasma concentration) are measured. The bioequivalence between both preparations can be shown according to a criterion of a guideline of generic products or the like.

### (4) Result

AUC₀₋₄₈ and Cmax of the test preparation and the standard preparation, the ratio of the geometric mean value of the test preparation relative to the standard preparation and 90% confidence interval are shown in Table 4.

As a result, these were within the criterion of bioequivalence (log(0.8) to log(1.25)) of a guideline for bioequivalence studies of generic products (Notification 0229 No.10 from the medicine food examination division, dated February 29, 2012) (a guideline of generic products), and it was confirmed that the test preparation was equal biologically to the standard preparation.

Furthermore, in the case of orally administrating the single dose of the test preparation with water, it was confirmed that it was equal biologically to the standard preparation in the same manner.

**[Table 4]**

| | Bioequivalence evaluation parameters | |
|---|---|---|
| | AUC₀₋₄₈(ng·hr/mL) | Cₘₐₓ (ng/mL) |
| Test preparation | 126.73±44.54 | 31.58±21.57 |
| Standard preparation | 115.08±33.72 | 2725±7.70 |
| The ratio of geometric mean value (Test preparation/Standard preparation) | 1.09 | 1.05 |
| 90%confidence interval | log(1.03) to log(1.14) | log(0.92) to log(1.21) |

| | | |
|---|---|---|
| (mean±standard deviation, n=25) | | |

### Example 1

Silodosin (100g), 300 g of D-mannitol (Mitsubishi Foodtech Co., Ltd.), 75 g of corn starch (Nihon Shokuhin Kako Co., Ltd.) and 25 g of talc (Matsumura Sangyo Co., Ltd.) were mixed using a fluidized bed granulation drier (MP-01, Powrex Corporation). A coating liquid prepared by adding 100 g of aminoalkyl methacrylate copolymer E (Evonik Degussa Japan Co., Ltd.), 10 g of sodium lauryl sulfate (Kao Corporation), 15 g of stearic acid (Mallinkrodt Co., Ltd.) and 35 g of talc (Matsumura Sangyo Co., Ltd.) to purified water was sprayed onto the mixture using a spray nozzle, and the mixture was granulated. The obtained granulated material was sized using a mill (P-02S, Dalton Co., Ltd.) with a screen size φ 0.55 mm to obtain masked particles (a-1).

A granulated material (b-1) was obtained using 948 g of D-mannitol (Mitsubishi Foodtech Co., Ltd), 41 g of corn starch (Nihon Shokuhin Kako Co.,Ltd) and 11 g of partially pregelatinized starch (Colorcon Nippon LLC) according to the common procedure.

Masked particles (a-1) (528 mg), 3101.8 mg of the granulated material (b-1), 280 mg of corn starch (Nihon Shokuhin Kako Co., Ltd.), 60 mg of calcium stearate (Nitto Kasei Kogyo K.K.) and 20 mg of light anhydrous silicic acid (Freund Corporation) were mixed to obtain granules comprising 4 mg of silodosin in a mass of 199.5 mg per unit preparation.

### Example 2

Silodosin (33.3 g), 366.7 g of D-mannitol (Mitsubishi Foodtech Co., Ltd), 75 g of corn starch (Nihon Shokuhin Kako Co., Ltd.) and 25 g of talc (Matsumura Sangyo Co., Ltd.) were mixed using a fluidized bed granulation drier (MP-01, Powrex Corporation).

A coating liquid prepared by adding 100 g of aminoalkyl methacrylate copolymer E (Evonik Degussa Japan Co., Ltd.), 10 g of sodium lauryl sulfate (Kao Corporation), 15 g of stearic acid (Mallinkrodt Co., Ltd) and 35 g of talc (Matsumura Sangyo Co., Ltd) to purified water was sprayed onto the mixture using a spray nozzle, and the mixture was granulated. The obtained granulated material was sized using a mill (P-02S, Dalton Co., Ltd) with a screen size φ 0.55 mm to obtain masked particles (a-2).

Masked particles (a-2) (792 mg), 1477 mg of the granulated material (b-1), 175 mg of corn starch (Nihon Shokuhin Kako Co., Ltd.), 37.5 mg of calcium stearate (Nitto Kasei Kogyo K.K.) and 12.5 mg of light anhydrous silicic acid (Freund Corporation) were mixed to obtain granules comprising 4 mg of silodosin in a mass of 249.4 mg per unit preparation.

### Example 3

Silodosin (100 g), 300 g of D-mannitol (Mitsubishi Foodtech Co., Ltd.), 75 g of corn starch (Nihon Shokuhin Kako Co., Ltd.) and 25 g of talc (Matsumura Sangyo Co., Ltd) were mixed using a fluidized bed granulation drier (MP-01, Powrex Corporation). A coating liquid prepared by adding 333.3 g of ethylcellulose aqueous dispersion having a solid content concentration of 30% (Asahi Kasei Chemicals Corporation) and 20 g of triethyl citrate (Wako Pure Chemical Industries, Ltd.) to purified water was sprayed onto the mixture using a spray nozzle, and the mixture was granulated. The obtained granulated material was sized using a mill (P-02S, Dalton Co., Ltd.) with a screen size φ 0.55 mm to obtain masked particles (a-3).

Masked particles (a-3) (496 mg), 3134.0 mg of the granulated material (b-1), 280 mg of corn starch (Nihon Shokuhin Kako Co., Ltd.), 60 mg of calcium stearate (Nitto Kasei Kogyo K.K.) and 20 mg of light anhydrous silicic acid (Freund Corporation) were mixed to obtain granules comprising 4 mg of silodosin in a mass of 199.5 mg per unit preparation.

### Example 4

Silodosin (120 g), 348 g of D-mannitol (Mitsubishi Foodtech Co., Ltd), 90 g of corn starch (Nihon Shokuhin Kako Co., Ltd.) and 30 g of talc (Matsumura Sangyo Co., Ltd.) were mixed using a fluidized bed granulation drier (MP-01, Powrex Corporation). The mixture was granulated while spraying a solution prepared by adding 12 g of hydroxypropylcellulose (Nippon Soda Co., Ltd.) to purified water using a spray nozzle. The obtained granulated material was sized using a mill (P-02S, Dalton Co., Ltd.) with a screen size φ 1.0 mm to obtain drug particles.

On the other hand, a coating liquid was obtained by adding 180 g of aminoalkyl methacrylate copolymer E (Evonik Degussa Japan Co., Ltd.), 18 g of sodium lauryl sulfate (Kao Corporation), 27 g of stearic acid (Mallinkrodt Co., Ltd.) and 63 g of talc (Matsumura Sangyo Co., Ltd.) to purified water.

Obtained drug particles (500 g) were charged into a fluidized bed granulation drier (MP-01, Powrex Corporation), and coated by spraying the coating liquid to obtain coated granules wherein the mass of aminoalkyl methacrylate copolymer E was 150 parts by mass relative to 100 parts by mass of silodosin. Obtained coated granules were sized using a mill (P-02S, Dalton Co., Ltd.) with a screen size φ 0.55 mm to obtain masked particles (a-4).

A granulated material (b-2) was obtained using 948 g of D-mannitol (Mitsubishi Foodtech Co., Ltd.) and 52 g of crospovidone (ISP Ltd) according to the common procedure.

Masked particles (a-4) (592 mg), 3038 mg of the granulated material (b-2), 280 mg of corn starch (Nihon Shokuhin Kako Co., Ltd.) and 80 mg of calcium stearate (Nitto Kasei Kogyo K.K.) were mixed to obtain a mixture for tableting. This mixture for tableting was tableted using a single punch tableting machine (N-30E, Okada Seiko) under the condition of a punch and die 8 mm, and a tableting pressure of about 5 kN to obtain a tablet of mass of 199.5 mg comprising 4 mg of silodosin per tablet.

### Example 5

Silodosin (100g), 300 g of D-mannitol (Mitsubishi Foodtech Co., Ltd.), 75 g of corn starch (Nihon Shokuhin Kako Co., Ltd.) and 25 g of talc (Matsumura Sangyo Co., Ltd.) were mixed using a fluidized bed granulation drier (MP-01, Powrex Corporation). A coating liquid prepared by adding 75 g of aminoalkyl methacrylate copolymer E (Evonik Degussa Japan Co., Ltd.), 7.5 g of sodium lauryl sulfate (Kao Corporation), 11.25 g of stearic acid (Mallinkrodt Co., Ltd.), 26.25 g of talc (Matsumura Sangyo Co., Ltd.) and 250 g of ethylcellulose aqueous dispersion having a solid content concentration of 30% (Asahi Kasei Chemicals Corporation) to purified water was sprayed onto the mixture using a spray nozzle, and the mixture was granulated. The obtained granulated material was sized using a mill (P-02S, Dalton Co., Ltd.) with a screen size φ 0.55 mm to obtain masked particles (a-5).

Masked particles (a-5) (556 mg), 3074 mg of the granulated material (b-2), 280 mg of corn starch (Nihon Shokuhin Kako Co., Ltd.), 60 mg of calcium stearate (Nitto Kasei Kogyo K.K) and 20 mg of light anhydrous silicic acid (Freund Corporation) were mixed to obtain a mixture for tableting. This mixture for tableting was tableted using a single punch tableting machine (N-30E, Okada Seiko) under the condition of a punch and die, 11×6 mm, and a tableting pressure of about 7 kN to obtain a tablet of mass of 199.5 mg comprising 4 mg of silodosin per tablet.

### Example 6

Silodosin (120 g), 348 g of D-mannitol (Mitsubishi Foodtech Co., Ltd.), 90 g of corn starch (Nihon Shokuhin Kako Co., Ltd.) and 30 g of talc (Matsumura Sangyo Co., Ltd) were mixed using a fluidized bed granulation drier (MP-01, Powrex Corporation). The mixture was granulated while spraying a solution prepared by adding 12 g of hydroxypropyl cellulose (Nippon Soda Co., Ltd.) to purified water using a spray nozzle. The obtained granulated material was sized using a mill (P-02S, Dalton Co., Ltd.) with a screen size φ 1.0 mm to obtain drug particles.

On the other hand, a coating liquid (c-1) was obtained by adding 180 g of aminoalkyl methacrylate copolymer E (Evonik Degussa Japan Co., Ltd.), 18 g of sodium lauryl sulfate (Kao Corporation), 27 g of stearic acid (Mallinkrodt Co., Ltd.), 63 g of talc (Matsumura Sangyo Co., Ltd.) to purified water.

Additionally, a coating liquid (c-2) was obtained by adding 50 g of D-mannitol (Mitsubishi Foodtech Co., Ltd.) to purified water.

Obtained drug particles (500 g) were charged into a fluidized bed granulation drier (MP-01, Powrex Corporation), and coated by spraying the coating liquid (c-1) to obtain coated granules wherein the mass of aminoalkyl methacrylate copolymer E was 150 parts by mass relative to 100 parts by mass of silodosin. Obtained coated granules were sized using a mill (P-02S, Dalton Co., Ltd.) with a screen size φ 0.55 mm to obtain masked particles.

Next, 500 g of obtained masked particles were charged into a fluidized bed granulation drier (MP-01, Powrex Corporation), and coated by spraying the coating liquid (c-2) to obtain coated granules wherein the mass of D-mannitol was 10 parts by mass relative to 100 parts by mass of masked particles. Obtained coated granules were sized using a mill (P-02S, Dalton Co., Ltd.) with a screen size φ 0.55 mm to obtain overcoated masked particles (a-6).

Overcoated masked particles (a-6) (976.8 mg), 4468.2 mg of granulated material (b-1), 420 mg of corn starch (Nihon Shokuhin Kako Co., Ltd.) and 120 mg of sodium stearyl fumarate (PHARMATRANS SANAQ AG) were mixed to obtain a mixture for tableting. This mixture for tableting was tableted using a single punch tableting machine (N-30E, Okada Seiko) under the condition of a punch and die, 8 mm, and a tableting pressure of about 5 kN to obtain a tablet of mass of 199.5 mg comprising 4 mg of silodosin per tablet.

### Example 7

Silodosin (120 g), 438 g of partially pregelatinized starch (Colorcon Nippon LLC) and 30 g of talc (Matsumura Sangyo Co., Ltd.) were mixed using a fluidized bed granulation drier (MP-01, Powrex Corporation). The mixture was granulated while spraying a solution prepared by adding 12 g of hydroxypropyl cellulose (Nippon Soda Co., Ltd.) to purified water using a spray nozzle. The obtained granulated material was sized using a mill (P-02S, Dalton Co., Ltd.) with a screen size φ 1.0 mm to obtain drug particles.

On the other hand, a coating liquid (c-3) was obtained by adding 240 g of aminoalkyl methacrylate copolymer E (Evonik Degussa Japan Co., Ltd.), 24 g of sodium lauryl sulfate (Kao Corporation), 36 g of stearic acid (Mallinkrodt Co., Ltd.), 84 g talc (Matsumura Sangyo Co., Ltd.) to purified water.

Obtained drug particles (500 g) were charged into a fluidized bed granulation drier (MP-01, Powrex Corporation), and coated by spraying the coating liquid (c-3) to obtain coated granules wherein the mass of aminoalkyl methacrylate copolymer E was 200 parts by mass relative to 100 parts by mass of silodosin. Obtained coated granules were sized using a mill (P-02S, Dalton Co., Ltd.) with a screen size φ 0.55 mm to obtain masked particles (a-7).

Masked particles (a-7) (984 mg), 4461 mg of the granulated material (b-1), 420 mg of corn starch (Nihon Shokuhin Kako Co., Ltd.) and 120 mg of sodium stearyl fumarate (PHARMATRANS SANAQ AG) were mixed to obtain a mixture for tableting. This mixture for tableting was tableted using a single punch tableting machine (N-30E, Okada Seiko) under the condition of a punch and die, 8 mm, and a tableting pressure of about 5 kN to obtain a tablet of mass of 199.5 mg comprising 4 mg of silodosin per tablet.

### Example 8

Silodosin (120 g), 441.6 g of pregelatinized starch (Nippon Starch Chemical Co., Ltd) and 30 g of talc (Matsumura Sangyo Co., Ltd.) were mixed using a fluidized bed granulation drier (MP-01, Powrex Corporation). The mixture was granulated while spraying a solution prepared by adding 8.4 g of hydroxypropylcellulose (Nippon Soda Co., Ltd.) to purified water using a spray nozzle. The obtained granulated material was sized using a mill (P-02S, Dalton Co., Ltd) with a screen size φ 1.0 mm to obtain drug particles.

Obtained drug particles (500 g) were charged into a fluidized bed granulation drier (MP-01, Powrex Corporation), and coated by spraying the coating liquid (c-3) to obtain masked particles wherein the mass of aminoalkyl methacrylate copolymer E was 200 parts by mass relative to 100 parts by mass of silodosin

Next, 500 g of obtained masked particles were charged into a fluidized bed granulation drier (MP-01, Powrex Corporation), and coated by spraying the coating liquid (c-2) to obtain coated granules wherein the mass of D-mannitol was 10 parts by mass relative to 100 parts by mass of masked particles. Obtained coated granules were sieved using a sieve of number 30 to obtain overcoated masked particles (a-8).

Overcoated masked particles (a-8) (721.6 mg), 2918.4 mg of granulated material (b-1), 280 mg of corn starch (Nihon Shokuhin Kako Co., Ltd.) and 80 mg of sodium stearyl fumarate (PHARMATRANS SANAQ AG) were mixed to obtain a mixture for tableting. This mixture for tableting was tableted using a single punch tableting machine (N-30E, Okada Seiko) under the condition of a punch and die, 8 mm, and a tableting pressure of about 7 kN to obtain a tablet of mass of 200.0 mg comprising 4 mg of silodosin per tablet.

### Example 9

Silodosin (4400 g), 16192 g of partially pregelatinized starch (Colorcon Nippon LLC) and 1100 g of talc (Matsumura Sangyo Co., Ltd.) were mixed using a fluidized bed granulation drier (NFLO-30SJC, Freund Corporation). The mixture was granulated while spraying a solution prepared by adding 308 g of hydroxypropylcellulose (Nippon Soda Co., Ltd.) to purified water using a spray nozzle. The obtained granulated material was sized using a mill (Millmeist, Freund Corporation) with a screen size φ 1.0 mm to obtain drug particles.

On the other hand, a coating liquid (c-4) was obtained by adding 6825 g of aminoalkyl methacrylate copolymer E (Evonik Degussa Japan Co., Ltd.), 682.5 g of sodium lauryl sulfate (Kao Corporation), 1023.8 g of stearic acid (Mallinkrodt Co., Ltd.) and 2388.8 g of talc (Matsumura Sangyo Co., Ltd) to purified water.

Additionally, a coating liquid (c-5) was obtained by adding 3000 g of D-mannitol (Mitsubishi Foodtech Co., Ltd.) to purified water.

Obtained drug particles (16250 g) were charged into a fluidized bed granulation drier (NFLO-30SJC, Freund Corporation), and coated by spraying the coating liquid (c-4) to obtain masked particles wherein the mass of aminoalkyl methacrylate copolymer E was 175 parts by mass relative to 100 parts by mass of silodosin. Next, obtained masked particles were coated by spraying the coating liquid (c-5) to obtain coated granules wherein the mass of D-mannitol was 10 parts by mass relative to 100 parts by mass of masked particles. Obtained coated granules were sieved using a sieve of number 30 to obtain overcoated masked particles (a-9).

A granulated material (b-3) was obtained using 19399 g of D-mannitol (Freund Corporation), 4095 g of microcrystalline cellulose (Asahi Kasei Chemicals Corporation) and 1706 g of crospovidone (ISP Ltd.) according to the common procedure.

Overcoated masked particles (a-9) (343 mg), 1477 mg of the granulated material (b-3), 140 mg of corn starch (Nihon Shokuhin Kako Co., Ltd.) and 40 mg of calcium stearate (Nitto Kasei Kogyo K.K) were mixed to obtain a mixture for tableting. This mixture for tableting was tableted using a single punch tableting machine (N-30E, Okada Seiko) under the condition of a punch and die, 8 mm, and a tableting pressure of about 6 kN to obtain a tablet of mass of 200.0 mg comprising 4 mg of silodosin per tablet.

### Example 10

Silodosin (4400 g), 16192 g of partially pregelatinized starch (Colorcon Nippon LLC) and 1100 g of talc (Matsumura Sangyo Co., Ltd.) were mixed using a fluidized bed granulation drier (NFLO-30SJC, Freund Corporation). The mixture was granulated while spraying a solution prepared by adding 308 g of hydroxypropylcellulose (Nippon Soda Co., Ltd.) to purified water using a spray nozzle. The obtained granulated material was sized using a mill (P-02S, Dalton Co., Ltd.) with a screen size φ 1.0 mm to obtain drug particles.

Obtained drug particles (16250 g) were charged into a fluidized bed granulation drier (NFLO-30SJC, Freund Corporation), and coated by spraying the coating liquid (c-4) to obtain masked particles wherein the mass of aminoalkyl methacrylate copolymer E was 175 parts by mass relative to 100 parts by mass of silodosin.

Next, obtained masked particles were coated by spraying the coating liquid (c-5) to obtain coated granules wherein the mass of D-mannitol was 10 parts by mass relative to 100 parts by mass of masked particles. Obtained coated granules were sieved using a sieve of number 30 to obtain overcoated masked particles (a-10).

A granulated material (b-4) was obtained using 19399 g of D-mannitol (Freund Corporation), 4095 g of microcrystalline cellulose (Asahi Kasei Chemicals Corporation) and 1706 g of crospovidone (ISP Ltd.) according to the common procedure.

Overcoated masked particles (a-10) (171.5 g), 738.5 g of the granulated material (b-4), 70 g of corn starch (Nihon Shokuhin Kako Co., Ltd.) and 20 g of sodium stearyl fumarate (PHARMATRANS SANAQ AG) were mixed to obtain a mixture for tableting. This mixture for tableting was tableted using a rotary tableting machine (CLEANPRESS Correct 12HUK, Kikusui Seisakusho Ltd.) under the condition of a punch and die, 8 mm, and a tableting pressure of about 7 kN to obtain a tablet of mass of 200.0 mg comprising 4 mg of silodosin per tablet.

### Example 11

Silodosin (120 g), 441.6 g of partially pregelatinized starch (National Starch & Chemical Ltd) and 30 g of talc (Matsumura Sangyo Co., Ltd.) were mixed using a fluidized bed granulation drier (MP-01, Powrex Corporation). The mixture was granulated while spraying a solution prepared by adding 8.4 g of hydroxypropyl cellulose (Nippon Soda Co., Ltd.) to purified water using a spray nozzle. The obtained granulated material was sized using a mill (P-02S, Dalton Co., Ltd.) with a screen size φ 1.0 mm to obtain drug particles.

Obtained drug particles (500 g) were charged into a fluidized bed granulation drier (MP-01, Powrex Corporation), and coated by spraying the coating liquid (c-3) to obtain masked particles wherein the mass of aminoalkyl methacrylate copolymer E was 200 parts by mass relative to 100 parts by mass of silodosin .

Next, obtained masked particles were charged into a fluidized bed granulation drier (MP-01, Powrex Corporation), and coated by spraying the coating liquid (c-2) to obtain coated granules wherein the mass of D-mannitol was 10 parts by mass relative to 100 parts by mass of masked particles. Obtained coated granules were sieved using a sieve of number 30 to obtain overcoated masked particles (a-11).

Overcoated masked particles (a-11) (721.6 mg), 2918.4 mg of the granulated material (b-1), 280 mg of corn starch (Nihon Shokuhin Kako Co., Ltd.) and 80 mg of sodium stearyl fumarate (PHARMATRANS SANAQ AG) were mixed to obtain a mixture for tableting. This mixture for tableting was tableted using a single punch tableting machine (N-30E, Okada Seiko) under the condition of a punch and die, 8 mm, and a tableting pressure of about 7 kN to obtain a tablet of mass of 200.0 mg comprising 4 mg of silodosin per tablet.

### Comparative Example 1

Silodosin (100 g), 300 g of D-mannitol (Mitsubishi Foodtech Co., Ltd.), 75 g of corn starch (Nihon Shokuhin Kako Co., Ltd.) and 25 g of talc (Matsumura Sangyo Co., Ltd.) were mixed using a fluidized bed granulation drier (MP-01, Powrex Corporation). A coating liquid prepared by mixing 166.7 g of vinyl acetate resin aqueous dispersion having a solid content concentration of 30%w/w (BASF Ltd.), 2.5 g of triethyl citrate (Wako Pure Chemical Industries, Ltd.) and 37.5 g of polyvinylpyrrolidone (ISP Ltd.) with purified water and further adding 25 g of talc (Matsumura Sangyo Co., Ltd.) was sprayed onto the mixture using a spray nozzle, and the mixture was granulated. The obtained granulated material was sized using a mill (P-02S, Dalton Co., Ltd.) with a screen size φ 0.55 mm to obtain masked particles (a-12).

Masked particles (a-12) (492 mg), 3138.0 mg of the granulated material (b-1), 280 mg of corn starch (Nihon Shokuhin Kako Co., Ltd.), 60 mg of calcium stearate (Nitto Kasei Kogyo K.K) and 20 mg of light anhydrous silicic acid (Freund Corporation) were mixed to obtain granules comprising 4 mg of silodosin in a mass of 199.5 mg per tablet.

The mean particle diameter of masked particles measured by Test Examples 1-7, and results of bitterness, time to start feeling bitterness, oral disintegration time, hardness and dissolution rate of granules and tablets comprising masked particles are shown in Table 5.

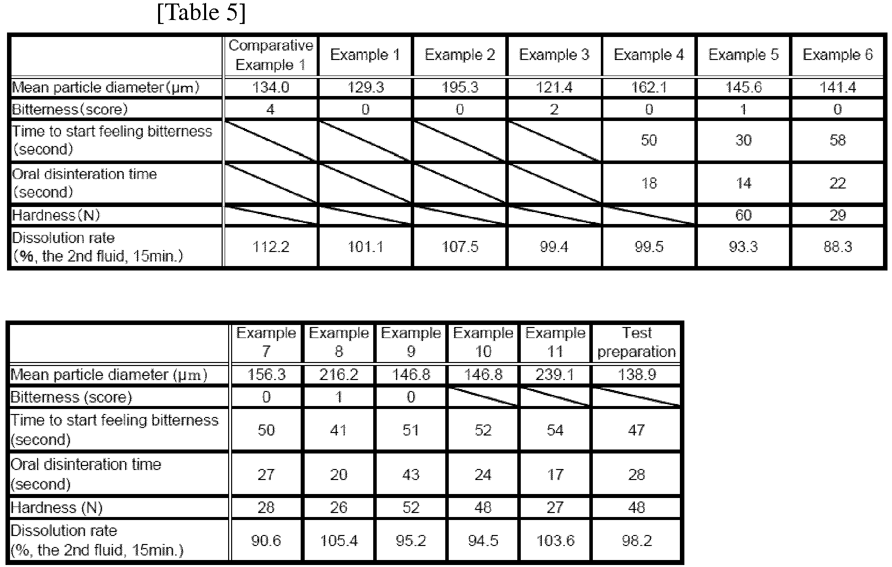

Each mass of silodosin, drug particles, non-enteric polymer and masked particles included in unit preparation and total mass of unit preparation are shown in Table 6.

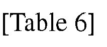

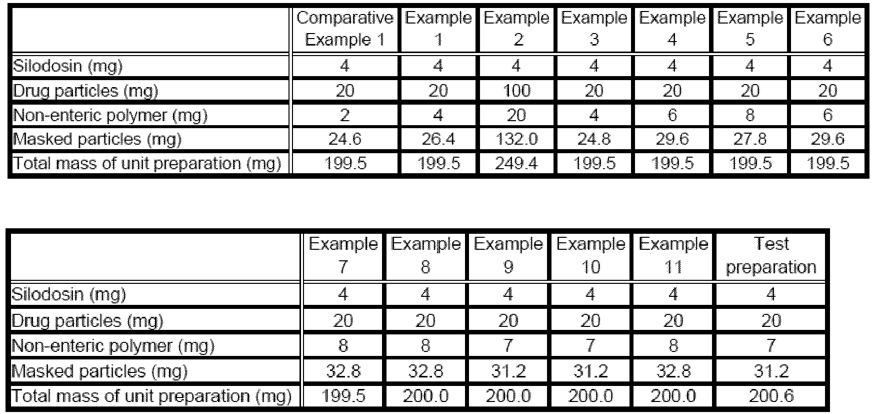

### Industrial Applicability

The present invention can provide a novel oral administration preparation that enables administration of silodosin, which is a drug with extremely strong bitterness, without a foreign-body sensation even without water, and has dissolution properties of being able to reproduce an effective blood concentration for the treatment of dysuria associated with benign prostatic hyperplasia or the like.

## Claims

1. A masked particle obtained by granulating or coating a drug particle comprising a fine powder of silodosin with a coating agent comprising a non-enteric polymer, wherein a content of the non-enteric polymer is 80 parts by mass to 400 parts by mass relative to 100 parts by mass of silodosin.

2. The masked particle as claimed in claim 1, wherein a dissolution rate after 15 minutes at pH 6.8 of an oral administration preparation comprising the masked particle is more than 85%.

3. The masked particle as claimed in claim 1 or 2, wherein a time to start feeling bitterness in human bitterness sensory test for an oral administration preparation comprising the masked particle is more than 30 seconds.

4. The masked particle as claimed in any one of claims 1 to 3, wherein the drug particle comprising a fine powder of silodosin is a mixture of silodosin and an additive.

5. The masked particle as claimed in claim 4, wherein the drug particle comprising a fine powder of silodosin is a granule of silodosin and an additive.

6. The masked particle as claimed in claim 4 or 5, wherein the additive is at least one additive selected from the group consisting of a sugar or a sugar alcohol and a starches.

7. The masked particle as claimed in any one of claims 1 to 6, wherein the non-enteric polymer is a gastro-soluble polymer.

8. The masked particle as claimed in any one of claims 1 to 6, wherein the non-enteric polymer is ethylcellulose, polyvinylacetal diethylaminoacetate or aminoalkyl methacrylate copolymer E.

9. The masked particle as claimed in any one of claims 1 to 8, wherein a content of the non-enteric polymer is 100 parts by mass to 200 parts by mass relative to 100 parts by mass of silodosin.

10. The masked particle as claimed in any one of claims 1 to 9, wherein a content of silodosin in the masked particle is 5 to 25 mass%.

11. The masked particle as claimed in any one of claims 1 to 10, wherein a content of the non-enteric polymer in the masked particle is 15 to 30 mass%.

12. The masked particle as claimed in any one of claims 1 to 10, wherein a content of the non-enteric polymer is 20 parts by mass to 40 parts by mass relative to 100 parts by mass of the drug particle.

13. An oral administration preparation comprising the masked particle as claimed in any one of claims 1 to 12.

14. The oral administration preparation comprising the masked particle as claimed in claim 13, wherein a dosage form is a tablet.

15. A method for the preparation of a masked particle, comprising the steps of:
(a) preparing a drug particle by mixing or granulating a fine powder of silodosin and an additive; and
(b) preparing a masked particle by granulating or coating a drug particle obtained by the step (a) with a coating agent comprising a non-enteric polymer, wherein a content of the non-enteric polymer is 80 parts by mass to 400 parts by mass relative to 100 parts by mass of silodosin.
